# EUROPEAN PATENT SPECIFICATION

(11) **EP 0 586 076 B1**
(45) Date of publication and mention of the grant of the patent: **25.06.2003**
(21) Application number: 93305833.1
(22) Date of filing: 23.07.1993
(51) Int. Cl.: C12N 15/86, C12N 15/49, A61K 39/21, C12N 7/01

(54) **Recombinant adenovirus vaccines**
Rekombinente Adenoviren-Impfstoffe
Vaccins adénoviraux recombinant

(30) Priority: 07.08.1992 US 926491
(43) Date of publication of application: 09.03.1994
(73) Proprietor: Wyeth, Madison, New Jersey 07940-0874 (US)
(72) Inventor: Davis, Alan Robert, Wayne, Pennsylvania 19087 (US); Hunt, Paul Porwen, Bryn Mawr, Pennsylvania 19010 (US); Lubeck, Micheal David, Glenmoore, Pennsylvania 19343 (US); Natuk, Robert James, Warrington, Pennsylvania 18976 (US); Chanda, Pranab Kumar, Wayne, Pennsylvania 19087 (US); Murthy, Shridhara Chikkatur Shankaranarayana, King of Prussia, Pennsylvania 19406 (US); Lee, Shaw-Guang Lin, Villanova, Pennsylvania 19085 (US)
(74) Representative: Connelly, Michael John

(56) References cited:
- WO-A-91/02804
- WO-A-92/11028
- WO-A-93/01297
- WO-A-93/11792
- US-A- 4 925 784
- JOURNAL OF INFECTIOUS DISEASES vol. 161 , 1990 pages 27 - 30 PREVEC, L. ET AL. 'A recombinant human adenovirus vaccine against rabies'
- NATUK ET AL PROC. NAT. ACD. SCI. USA vol. 89, August 1992, USA, pages 7777 - 7781
- NATURE MEDICINE vol. 3, no. 6, March 1997, pages 651 - 658

## Description

### BACKGROUND OF THE INVENTION

A major goal of biomedical research is to provide protection against viral disease through immunization. One approach has been to use killed vaccines. However, large quantities of material are required for killed vaccine in order to retain sufficient antigenic mass. In addition, killed vaccines are often contaminated with undesirable products during their preparation. Heterologous live vaccines, using appropriately engineered adenovirus, which is itself a vaccine, seems like an excellent immunogen [Chanock R., JAMA, 195, 151 (1967)]. Our invention concerns vaccines using adenovirus as a vector.

Presently marketed adenovaccine comprises live, infectious adenoviruses in an enteric-coaled dosage form. Upon administration to the patient to be vaccinated, the virus is carried past the upper-respiratory system (where disease-producing infection is thought to occur), and is released in the intestine. In the intestine, the virus reproduces in the gut wall, where, although it is not capable of causing adenoviral disease, nevertheless induces the formation of adenovirus antibodies, thus conferring immunity to adenoviral disease. In our invention, live, infectious adenovirus which has been engineered to contain genes coding for antigens produced by other disease-causing organisms. Upon release the virus will reproduce and separately express both the adenoviral antigen and the pathogen antigen, thereby inducing the formation of antibodies or induce cell mediated immunity to both adenovirus and the other disease-causing organism. By "live virus" is meant, in contradistinction to "killed" virus, a virus which is, either by itself or in conjunction with additional genetic material, capable of producing identical progeny. By "infectious" is meant having the capability to deliver the viral genome into cells.

Roy, in European Patent Publication 80,806 (1983), proposed a method for producing immunity to microbial diseases by the administration of a microbe containing a foreign gene which will express an antigen of a second microbe to which immunity is conferred. He states that preferred oral preparations are enteric coated. Dubelcco proposed recombinant adenovirus vaccines in which the surface protein of adenovirus is modified to contain in its structure a segment of foreign protein which will produce a desired biological response on administration to animals. [PCT International Publication WO 83/02393 (1983)]. Davis discloses oral vaccines derived from recombinant adenoviruses. [UK Patent GB 2166349 B].

Human immunodeficiency virus type 1 (HIV-1) has been etiologically associated with acquired immunodeficiency syndrome (AIDS) and related disorders. [Barre-Sinoussi, F., Science 220: 868 (1983); Gallo, R., Science 224: 500 (1984); Popovic, M., Science 224: 497 (1984); Sarngadharan, M., Science 224: 506 (1984)]. AIDS is now a worldwide epidemic for which, currently, there is no vaccine or cure. Most of the effort for vaccine development has focused on the envelope (env) glycoprotein as an antigen which might provide protective immunity. Antisera prepared against purified gp 120 can neutralize HIV-1 in vitro. [Crowl, R., Cell 41: 979 (1985); Putney, S., Science 234: 1392 (1986); Ho, D., J. Virol. 61: 2024 (1987); Nara, P., Proc. Natl. Acad. Sci. USA 84: 3797 (1987)]. HIV-1 envelope antigen has been produced in different expression systems including Escherichia coli [Crowl, R., Cell 41: 979 (1985); Chang, T., Bio/Technology 3: 905 (1985); Dawson, G., J. Infect. Dis. 157: 149 (1988)] as well as mammalian [Chakrabarti, S., Nature 320: 535 (1986); Dewar, R., J. Virol. 63: 129 (1989); Rekosh, D., Proc. Natl. Acad. Sci. USA 85: 334 (1988); Whealy, M., J. Virol. 62: 4185 (1988)] yeast [Barr, P., Vaccine 5: 90 (1987)] and insect cells [Hu, S., Nature 328: 721 (1978); Rusche, J., Proc. Natl. Acad. Sci. USA 84: 6294 (1987)].

Live recombinant vaccinia virus expressing the entire HIV-1 env glycoprotein [Hu, S., J. Virol. 61: 3617 (1987)] or purified recombinant gp 120 env glycoprotein [Berman, P., Proc. Natl. Acad. Sci. USA 85: 5200 (1988)] were evaluated in chimpanzees as vaccine candidates. Active immunization with these vaccines induced a good cell-mediated immune response as well as cytotoxic T-cell activity to the env antigen [Zarling, J., J. Immunol. 139: 988 (1987)]. All experimental animals seroconverted as assayed by ELISA and Western blotting. However, immunized chimpanzees developed no or only low titers of neutralizing antibody to HIV-1. Challenge with live virus failed to protect chimpanzees against these vaccines. Type-specific HIV-1 neutralizing antibodies were found in chimpanzees early in infection against a variable domain (V3) within the C-terminus half of gp 120 [Goudsmit, J., Proc. Natl. Acad. Sci. USA 85: 4478 (1988)]. The recombinant gp 120 made in insect cells has also been shown to induce humoral immune response in goat (RuscheJ., Proc. Natl. Acad. Sci. USA 84: 6294 (1987)]. Zagury [Nature 332: 728 (1988)] have demonstrated both anamnestic humoral and cellular immune reaction in humans using a vaccine virus recombinant expressing gp 160. [Chakrabarti, S., Nature 320: 535 (1986); Hahn, B., Proc. Natl. Acad. Sci. USA 82: 4813 (1985)]. Both group-specific cell-mediated immunity and cell-mediated cytotoxicity against infected T4 cells were also found. These results indicate that an immune state against HIV-1 can be obtained in humans using recombinant env-based vaccine. Recently, Desrosiers has shown that vaccination with inactivated whole simian immunodeficiency virus (SIV) can protect macaques against challenge with live SIV. [Proc. Natl. Acad. Sci. USA 86: 6353 (1989)]. These data provide hope that vaccine protection against human AIDS virus, HIV-1, infection may be possible.

Chanda discloses high level expression of the envelope glycoproteins of HIV-1 in the presence of rev gene using helper-independent adenovirus type 7 recombinants. [Virology 175: 535 (1990)]. Vemon discloses the ultrastructural characterization of HIV-1 gag-containing particles assembled in a recombinant adenovirus vector system. [J. Gen. Virology 72: 1243 (1991)]. Vernon also discloses the preparation of the HIV-1 recombinant adenoviruses Ad7-rev-gag and Ad4-revgag.

The present invention provides use of a live recombinant adenovirus to prepare a medicament for use in treatment to produce antibodies or cell mediated immunity to human immunity virus type 1, in which the virion structural protein of the live recombinant adenovirus is unchanged from that in the native adenovirus from which the recombinant adenovirus is produced, and which contains the gene coding for an antigen corresponding to the said antibodies or inducing the said cell mediated immunity, wherein the recombinant adenovirus is selected from one or more of the group consisting of:
Ad7-tplenv-tplHrev, Ad7-tplgag-tplHrev, Ad7-rev-gag, Ad4-tplenv-tplHrev, Ad4-tplgag-tplHrev, Ad4-rev-gag, Ad5-tplenv-tplHrev, and Ad5-tplgag-tplHrev,
the medicament being for intranasal administration to a mammal of the order Primates including man

In a further embodiment of the invention the medicament further comprises one or more than one human immunodeficiency type 1 subunit protein. Preferably the subunit protein is env or gag

While the specification specifically refers to adenoviruses having an early region 3 (E3) deletion, adenoviruses which are attenuated, contain a temperature sensitive lesion, or a E1 deletion may also be used as a vector.

In a preferred embodiment, the treatment includes administering the recombinant adenovirus both prophylactically to an HIV-1 susceptible mammal and as immunotherapy following detection of HIV in said mammal. Regimens containing the following recombinant adenoviruses were used to produce the anti-HIV responses.

| Virus Name | Descriptive Name | ATCC Name |
|---|---|---|
| Ad7-env | Ad7-tplenv-tplHrev | VR-2299 |
| Ad7-gag | Ad7-tplgag-tplHrev | VR-2393 |
| Ad7-gag-1 | Ad7-rev-gag | VR-2392 |
| Ad4-env | Ad4-tplenv-tplHrev | VR-2293 |
| Ad4-gag | Ad4-tplgag-tplHrev | VR-2391 |
| Ad4-gag-1 | Ad4-rev-gag | VR-2390 |
| Ad5-env | Ad5-tplenv-tplHrev | VR-2297 |
| Ad5-gag | Ad5-tplgag-tplHrev | VR-2298 |

Referring to the above table Ad4, Ad5, and Ad7 refer to human adenoviruses types 4, 5, and 7 respectively in which the E3 region has been deleted. Env refers to the HIV envelope glycoprotein (gp 160) gene. Gag refers to the HIV gag/pro gene. Rev refers to the HIV regulatory gene. Hrev refers to an altered version of the rev gene where the nucleotide sequences were changed without changing the aminoacid sequence employing codons that were frequently used in human genes. Tpl refers to the upstream adenovirus tripartite leader sequence with an intervening sequence between the first and second leaders that are positioned in front of the recombinant genes.

In further embodiments of the present invention there is provided live recombinant viruses X-tplY-tplHrev in which X is Ad4, Ad5 or Ad7 and Y is env or gag.

As described in detail below, intranasal administration of Ad-HIV recombinant viruses to naive chimpanzees resulted in both priming and boosting of both humoral and cell-mediated immune responses directed at HIV recombinant antigens. The recombinant adenoviruses administered to chimpanzees were shown to produce antibodies to the env and gag proteins of HIV. IgG antibodies specific for HIV were observed in nasal, saliva, and vaginal secretions following administration of the recombinant adenoviruses and IgA antibodies specific for HIV were observed in nasal and saliva secretions. The first set of recombinant viruses (Ad7) appeared to be shed the longest period of time and induce the best anti-Ad antibody response. The results also showed that administration of Ad-HIV vaccines by the intranasal route was superior to administration of enteric-coated recombinant viruses by the oral route.

Optimum immune responses directed at HIV antigens required primary infection one booster immunization with a heterotypic recombinant Ad-HIV to elicit strong anti-HIV binding antibodies. Intranasal administration of the Ad-HIV viruses effectively primed chimpanzees to respond with high titered neutralizing antibodies to HIV-1 following subsequent HIV-1 subunit protein booster immunization.

### Preparation of Representative Recombinant Adenoviruses

The following Examples show the construction of representative recombinant adenoviruses of this invention. The recombinant viruses were propagated on A549 cells and subsequently titered on A549 cells.

### Example 1. Ad7-gag-1

The construction of recombinant adenoviruses containing the gene for the HIV envelope protein has been described [Chanda, P., Virology 175: 535 (1990)]; a similar procedure was used to incorporate gag and pro [see Vernon, S., J. Gen. Virology 72: 1243 (1991)]. Briefly, a DNA fragment containing the entire gag and pro coding regions (bp 335 to 2165) of HIV-1 strain LAV [Wain-Hobson, S., Cell 40: 9, (1985)] was constructed with a unique Sa/I site in front of the AUG codon of the gag gene and an XbaI site at bp 2165, for the insertion of the viral rev-responsive element (rre; bp 7178 to 7698). A 2.37 kb SaI fragment containing the three HIV-1 sequences was inserted at a SaI site in an expression cassette containing the adenovirus type 7 (Ad7) major late promoter (MLP), the tripartite leader (TPL) with an intervening sequence between the first and second leaders, and the hexon polyadenylation site (poly A) as described in Chanda [Virology 175: 535 (1990)]. The cassette was inserted 159 bp from the right end of an Ad7 genome [Sussenbach, The Adenoviruses, Ginsberg, ed., Plenum Press, pp. 34-124 (1984)] containing the HIV-1 rev gene [Feinberg, M., Cell 46: 807 (1986); Sodroski, J., Nature 321: 412 (1986)] in a deleted [79.5 to 88.4 map units (m.u.)] E3 region [Chanda, P., Virology 175: 535 (1990)].

### Example 2. Ad4-gag-1

Following the procedure for the construction of the Ad7-gag-1 recombinant adenovirus in Example 1, a similar expression cassette containing analogous Ad4 sequences and the three HIV coding regions were inserted at a site 139 bp from the right end of an Ad4 genome which contained HIV-1 rev in an E3 deletion between 76 and 86 m.u.

### Example 3. Ad5-eny

Ad5-tplenv-tplHrev contains the entire coding sequence of HIV-1 (LAV strain) gp160 and a modified version of the rev gene, called Hrev. Both the env as well as rev gene are preceded by a synthetic copy of the Ad5 tripartite leader (Ad5-tpl). Ad5-tpl was chemically synthesized and was cloned in pTZ vector. Then the gp160 DNA sequence was inserted behind the Ad5-tpl to create Ad5-tplenv/PTZ18R clone. The Hrev (∼360bp) was also chemically synthesized where the nucleotide sequences were changed without changing the amino acid sequence with the help of the codon usage. This was done to avoid homologous recombination as some identical sequences exist between env and rev. In an analogous way like Ad5-tplenv construct, Hrev gene was also inserted behind tpl in pTZ18R vector to create the plasmid, Ad5-tplHrev. The entire sequence containing Ad5-tplHrev was excised out and then inserted behind Ad5-tplenv to create the plasmid, Ad5-tplenv-tplHrev. This plasmid was then inserted in the deleted E3 region of Ad5 Marietta strain (78.8-85.7 mu deletion) at 78.8 mu. This plasmid was linearized with BglI enzyme and then mixed with 0-87 mu SnaB1 fragment that was derived from the wild-type purified Ad5 virus. After A549 cells were transfected with the DNA mixtures, recombinant virus plaques were picked, plaque purified three times, and their genomic structures were confirmed by restriction endonuclease site analysis of DNA extracted from infected cells by the method of Hirt. [J. Mol Bio. 26: 365 (1967)].

### Example 4. Ad5-gag

Ad5-tplgag-tplHrev contains the entire gag and pro region as well as the modified rev gene, Hrev. A copy of the Ad5 synthetic tripartite leader was placed in front of the gag and Hrev genes. A DNA fragment containing the entire gag and pro regions (bp 335 to 2165 of LAV strain of HIV-1) was constructed with a unique Sail site in front of AUG codon of the gag gene and an xba site at bp 2165, for the insertion of the viral rev-responsive element (rre; bp 7178-7698). Two separate plasmids Ad5-tplgag as well as Ad5-tplHrev were constructed in a similar way as described for Ad5-tplenvtplHrev. Then the Ad5-tplHrev fragment was inserted behind Ad5-tplgag to create the plasmid Ad5-tplgag-tplHrev. Then the fragment Ad5-tplgag-tplHrev was inserted at the unique XbaI site at map position 78.8 of the Ad5 Marietta strain with an E3 deletion (78.8-85.7 mu E3 deletion). Then the final plasmid containing the Ad5 sequence was linearized and then mixed with the 0-87 mu SnaB1 viral fragment for transfection. Recombinant plaques were picked up, plaque purified three times, and were checked by Hirt analysis of DNA extracted from the infected cells.

### Example 5. Enteric Coated Capsules

Recombinant adenoviruses were grown in A549 cells and harvested following 3 cycles of freeze-thawing. Clarified infected cell lysates were lypholized and 60 to 100 mg were packed into #2 gelatin capsules using a 1 ml syringe plunger under dehumidified conditions. The capsules were coated with a 10% cellulose acetate phthalate in acetone/100% Ethanol (1:1) by manually dipping each end 6 times with air drying between dips. A coating between 69 to 77 mg of cellulose acetate phthalate was formed under these conditions. Sample capsules were tested for resistance to simulated gastric fluid (0.32% pepsin, 0.2% NaCI pH 1.2) at 37 C using a VanKel Disintegration Testor apparatus for 1 hr. The capsules were inspected for holes or cracks and transferred to a 15 ml tube containing 10 ml of simulated intestinal fluid (1.0% pancreatin, 0.05 M monobasic potassium phosphate pH 7.5) and rotated at 37° C. All capsules tested were resistant to simulated gastric fluid for 1 hr at 37 C with agitation and began to dissolve within 15 min. in simulated intestinal fluid. The amount of virus was titrated on confluent A549 cell monolayers by a plaque assay and the viral DNA stability confirmed by Hirt analysis.

### Treatment Regimens

Immunogenicity of the recombinant adenoviruses for HIV was evaluated in chimpanzees under three treatment regimens. The first regimen consisted of administering the recombinant adenovirus orally via an enterically coated capsule (Example 5) at 0, 7, and 26 weeks followed by an env + gag subunit protein booster using alum as an adjuvant. The second regimen consisted of further treating the chimpanzees that received regimen 1 at 46 and 58 weeks with additional boosters of recombinant adenovirus administered intranasally. The third treatment regimen consisted of administering recombinant adenovirus intranasally to naive chimpanzees at weeks 0, 24, and 52 followed by an env subunit booster at week 72.

The following table summarizes treatment regimens 1 and 2.

| **TREATMENT REGIMENS 1 AND 2** | | | |
|---|---|---|---|
| **Immunization** | **Time** | **Chimpanzees 1 and 2** | **Chimpanzee 3** |
| **Regimen 1** | | | |
| Primary* | 0 weeks | 1.5 x 10⁷ pfu Ad7-env 2.0 x 10⁹ pfu Ad7-gag-1 | 1.5 x 10⁷ pfu Ad7-env |
| 1st Booster* | 7 weeks | 1.1 x 10¹⁰ pfu Ad4-env 1.0 x 10¹⁰ pfu Ad4-gag-1 | 1.1 x 10¹⁰ pfu Ad4-env |
| 2nd Booster* | 26 weeks | 7.9 x 10¹⁰ pfu Ad5-env | 7.9 x 10¹⁰ pfu Ad5-env |
| 3rd Booster⁺ | 34 weeks | 200 ug env in 0.2% alum 500 ug env in 0.2% alum | 200 µg env in 0.2% alum |

| **Regimen 2** | | | |
|---|---|---|---|
| 1st Intranasal Boost | 46 weeks | 1.0 x 10⁸ pfu Ad7-env 1.0 x 10⁸ pfu Ad7-gag | 1.0 x 10⁸ pfu Ad7-env |
| 2nd Intranasal Boost | 58 weeks | 1.0 x 10⁸ pfu Ad4-env 1.0 x 10⁸ pfu Ad4-gag | 1.0 x 10⁸ pfu Ad4-env |

| | | | |
|---|---|---|---|
| *Each dose was administered in enteric-coated gelatin capsules on 3 consecutive days. | | | |
| ⁺Administered intramuscularly. | | | |

The following table summarizes treatment regimen 3.

| **TREATMENT REGIMEN 3** | | | |
|---|---|---|---|
| **Immunization** | **Time** | **Chimpanzees 4 and 5** | **Chimpanzee 6** |
| Primary* | 0 weeks | 1.0 x 10⁷ pfu Ad7-env 1.0 x 10⁷ pfu Ad7-gag | 1.5 x 10⁷ pfu Ad7-env |
| 1st Booster* | 24 weeks | 1.0 x 10⁷ pfu Ad4-env 1.0 x 10⁷ pfu Ad4-gag | 1.5 x 10⁷ pfu Ad4-env |
| 2rd Booster* | 52 weeks | 1.0 x 10⁷ pfu Ad5-env 1.0 x 10⁷ pfu Ad5-gag | 1.5 x 10⁷ pfu Ad5-env |
| 3rd Booster⁺ | 75 weeks | 0.5 mg env | |

| | | | |
|---|---|---|---|
| *Administered intranasally. | | | |
| ⁺Administered intramuscularly. | | | |

### Measurement of Immunogenicity: Treatment Regimen 1

### Chimpanzee Inoculations

Three chimpanzees (2 males and 1 female) that were screened negative for the presence of neutralizing antibodies to human adenoviruses type 4, and 7 were evaluated using treatment regimen 1. Enteric-coated capsules containing recombinant adenoviruses were given using a stomach tube under anesthesia on three consecutive days. Two chimpanzees (1 and 2) received both env and gag recombinant viruses while the third chimp (3) received only env recombinant viruses.

Adenovirus-derived subunit preparations containing env or gag gene products were purified from infected A549 cell cultures [see Vernon, S., J. Gen. Virology 72: 1243 (1991) and Natuk, R., Proc. Natl. Acad. Sci. USA 89: 7777 (1992)]. Recombinant antigens were formulated with alum adjuvant and administered intramuscularly, 200 ug/dose env and 500 ug/dose gag particles.

Whole blood, serum, and stool samples were collected at different times during the course of the experiment. Whole blood was processed to obtain white blood cell populations for FACS, HIV CTL (using recombinant vaccinia viruses expressing HIV-env, HIV-gag, or the lac gene products), and for lymphoproliferative assays to purified HIV recombinant gp160, gp120, and p24. Serum and stool specimens were stored at -70°C C until use.

### Detection of Recombinant Adenoviruses in Stool Specimens

Chimpanzee stool specimens were thawed and 10% (V/V) suspensions were made into antibiotic containing DMEM. Clarified stool suspensions were used to infect confluent A549 cell monolayers in 60 mm tissue culture dishes. After a 1 hr adsorption period the unbound material was washed away and the monolayers were overlaid with an 0.5% agar overlay medium. Plaques were allowed to develop for 7-10 days and plaques were visualized by neutral red staining, counted and the agar overlay was gently removed taking care not to disturb the cell monolayer. The cell sheet was transferred to nitrocellulose filter membranes (Millipore Type HA, 0.45 um), presoaked in 20X SSC and placed on the cell layer and left in contact with the cell monolayer for 2 to 4 minutes. The filters were peeled off, air-dried, and baked for 2 hr in a vacuum oven at 80° C. Nitrocellulose filters were washed twice in 3X SSc/0.1% SDS at room temperature and prehybridized and hybridized according to standard procedures [Poncet, D., J. Virol. Methods 26: 27 (1989)]. ³²P-labeled oligo-probes were added to the hybridization buffer (1 x 10⁶ CPM) and incubated overnight at 42° C. DNA probes were prepared by which could detect either Ad4 fiber, Ad5 fiber, Ad7 fiber, HIV-env or HIV-gag specific sequences. [Wain-Hobson, Cell 40: 9 (1985)]. The filters were washed, autoradiographed, and hybridization signals were counted.

### Adenovirus Neutralization Test Procedures

Serial 2-fold dilutions (starting with 1:4) of heat-inactivated (56 C fdor 30 min.) dog serum were made in 96-well microtiter plates (0.05 ml/well) and were mixed with 0.05 ml media containing 30-100 TCID₅₀ virus for 1 hr at 37° C. To each well 0.05 ml of media containing 2x 10⁴ A549 cells were added and the plates were incubated at 37° C 5% CO₂ for 7-10 days. All samples were done in duplicate. Virus and uninfected cell controls were included in each assay for determining the end point in test sera. Titers were expressed as the reciprocal of the lowest dilution at which 50% cytopathic effect was observed.

### Detection of Anti-HIV Antibodies by ELISA and Western Blotting

Detection of anti-HIV antibodies Chimpanzee antibody responses to HIV-1 antigens were measured by testing various dilutions by commercial ELISA and Western blot kits as instructed by the manufacturers (DuPont, Wilmington, DE).

### Results

Feces were collected from each chimpanzee prior to and after virus inoculation and stored at -70° C. Ten percent suspensions were prepared from each sample and were used to infect confluent A549 cell monolayers. After 7-10 days viral plaques were identified by neutral red staining and the cell monolayers were transferred to nitrocellulose membranes. Representative samples were hybridized with various labeled oligo-probes to detect sequences specific for Ad4, Ad5, Ad7, HIV-env, or HIV-gag genes. Identification of specific recombinant Ad-HIV viruses could be determined by this plaque hybridization technique. None of the recombinant viruses were shed into the feces for longer than 7 days p.i. Peak titers were always associated with 1-3 day samples and most likely represented the non-adsorbed virus inoculum. Previous chimp studies using Ad-HBsAg recombinants had indicated that Ad-HBsAg recombinants could be detected for 30-40 days p.i. With the enteric capsule route of administration, it appeared that these recombinant viruses did not replicate well in vivo.

Seroconversion to the serotype of the adenovirus vectors employed was determined by neutralization test procedures. Very low to modest anti-adenovirus serum titers were measured to all 3 serotypes used in each of the chimpanzees.

Seroconversion to recombinant HIV gene products were determined by either ELISA or Western blotting techniques. No ELISA response was detected in any of the chimpanzees prior to the second booster inoculation with the Ad5-env recombinant. Two weeks following Ad5-env inoculation anti-env responses could be measured in 2 of the 3 animals. Intramuscular injection of gag and/or env preparations had a slight boosting effect in 1 of the 3 animals. Western blot analysis appeared to be much more sensitive than the ELISA and had the further advantage of identification of which env and/or gag gene products were being recognized as being immunogenic. Low serum antibody titers were measured following both the primary Ad7 recombinant and first booster with Ad4 recombinants viruses. A significant increase in serum titer to env gene products was observed following the second booster immunization with the Ad5-env recombinant. Significant increases in the 2 animals which received gag gene products were seen following injection with subunit preparations. Despite relatively good Western blot titers to HIV antigens, only 1 of the 3 animals responded with serum neutralizing antibodies. This response in chimpanzee 2 was very low (titer of 10 to 20).

These results are summarized in the following table.

| **RESULTS OBTAINED USING TREATMENT REGIMEN 1** | | | | | | |
|---|---|---|---|---|---|---|
| Chimp Number | Recombinant Virus | Recombinant Virus Shedding Stools (Days) | Peak Anti-Adeno Neutralizing Titer | Western Blot Peak anti-HIV Titers | | Peak Anti-HIV Neutralizing Titer |
| | | | | env | gag | |
| 1 | Ad7-env, Ad7-gag-1 Ad4-env, Ad4-gag-1 Ad5-env subunit: env + gag | 2,2 2,2 7+ | 128 8 128 | - - 100 100 | 20 20 - 1000 | <10 <10 <10 <10 |
| 2 | Ad7-env, Ad7-gag-1 Ad4-env, Ad4-gag-1 Ad5-env subunit: env + gag | 3,2 1,7 7+ | 64 128 64 | - 20 10000 1000 | 20 100 - 10000 | <10 <10 20 10 |
| 3 | Ad7-env Ad4-env Ad5-env subunit: env | 2 1 7+ | 6 128 512 | 20 20 1000 100 | N/A* N/A N/A N/A | <10 <10 <10 <10 |

| | | | | | | |
|---|---|---|---|---|---|---|
| *N/A = not applicable. | | | | | | |

Cell-mediated immunity was measured in peripheral blood mononuclear cell population obtained from chimpanzees. HIV specific CTL activity was measured by determining lysis of syngenic target cells that were infected with vaccinia virus recombinants that express either the HIV-env gene products, the HIV-gag gene products, or the lac gene product (control for nonspecific cytotoxicity). A hint of HIV specific CTL-like activity was measured in this way.

Lymphoproliferative assays were performed to determine whether purified recombinant env (gp160, gp120) or gag (p24) preparations were capable of stimulating blastogenesis. No proliferation was measured after the primary inoculum and only 1 of the 3 animals show a lymphoproliferative response following administration of the first boost with Ad4 recombinant viruses. All 3 animals responded with proliferative responses after the second booster (Ad5-env) and the third boost (subunit preparations).

### Measurement of Immunogenicity; Treatment Regimen 2

### Chimpanzee Inoculations and Collection of Data

Three chimpanzees (2 males and 1 female) that were previously inoculated with Ad-HIV recombinant viruses in enteric-coated capsules and boosted with adenovirus-derived gag and/or env subunits (treatment regimen 1) were infected intranasally with Ad7-HIV viruses (week 46) and 12 weeks later (week 58) with Ad4-HIV viruses. Recombinant adenoviruses were given in tissue culture media diluted with phosphate saline buffer dropwise into the nostrils of chimpanzees under anesthesia. Two chimpanzees (numbers 1 and 2) received both env and gag recombinant viruses while the third chimp (number 3) received only env recombinant viruses.

Whole blood, serum, and stool samples were collected at different times during the course of the experiment, and processed as described in Regimen 1. Adenovirus detection in stool samples or nasal swabs, adenovirus neutralization test procedures, and detection of anti-HIV antibodies were performed according to the procedures described in Regimen 1.

### Results

The first intranasal booster with Ad7 recombinants was given in one dose of 1x10⁸ pfu's/chimpanzee. At the time of virus administration chimpanzees 3, 1, and 2 had serum anti-Ad7 neutralization titers of <4, 8, and 64 respectively from previous oral immunizations. Nasal swabs and stool samples were examined for the presence of shed recombinant viruses by a plaque hybridization technique. Recombinant Ad7-env was detected in nasal swabs up to 7 days p.i. in two of the animals. Recombinant Ad7-env and Ad7-gag were found to be present in stool samples from 5 to 12 days p.i. There was a correlation between the serum titer to Ad7 and the ability to detect recombinant viruses in nasal swabs and stool specimens. The two animals which displayed marginal anti-HIV antibody response were greatly augmented by the intranasal boost. The third animal was boosted to a lesser extent. Low titered neutralizing antibodies directed at HIV could now be detected in all three animals. Secretory antibodies were detected in nasal swab specimens which contained anti-gag and/or env binding antibodies. No signs or symptoms of respiratory disease were observed in these animals as a result of intranasal administration of the Ad7 recombinant viruses.

Three months later these chimpanzees were immunized with Ad4 recombinants at a single dose of 1x10⁸ pfu's/chimpanzee/virus. These animals had serum anti-Ad4 neutralization titers between 128 to 256 from previous oral immunization at the time of intranasal challenge. At 3 days post-infection 2 of the animals (2 and 3) had a slight cough. The third animal (number 1) died on day 5 from a bacterial pneumonia (Streptococcus pneumoniae was isolated). The other two animals presented harsh sounds by auscultation and S. pneumoniae was isolated from both chimpanzees. Antibiotic treatments were initiated and both chimpanzees recovered.

Upon retrospective examination of this situation several observations could be made. At the time of intranasal administration chimpanzee number 1 was already experiencing a fever and an abnormal Complete Blood Count. There was a disproportionate number of polymorphonuclear cells present and a 5% level of band cells (immature polymorphonuclear cells) taken together, this information indicated that there was a significant bacterial infection taking place prior to virus inoculation. Autopsy specimens taken from the lung, liver, spleen, and serum all tested negative for the presence of infectious adenovirus by tissue culture using 3 blind passages on susceptible A549 cell monolayers. Similar findings were obtained by plaque hybridization techniques. Lung and liver paraffin embedded samples tested negative for the presence of adenovirus antigens using a commercial immunofluorescent kit for adenovirus antigens. Inclusion bodies were observed in H&E stained lung sections. There was a disagreement by experts as to whether these inclusions were caused by adenovirus or not. Several weeks later another chimpanzee experienced a similar fate at the same primate center and died. While it was likely that administration of recombinant adenoviruses had a only a minor role, if any, in causing the death of chimpanzee number 1 it was considered prudent to administer antibiotics prophylactically prior to and after any further intranasal administration of adenovirus recombinants to chimpanzees.

The following table shows the results obtained using treatment Regimen 1 and the Ad7-recombinants in Regimen 2.

| **RESULTS OBTAINED USING TREATMENT REGIMENS 1 AND 2** | | | | | | |
|---|---|---|---|---|---|---|
| Chimp Number | Recombinant Virus | Recombinant Virus Shedding Stools (Days) | Peak Anti-Adeno Neutralizing Titer | Western Blot Peak anti-HIV Titers | | Peak Anti-HIV Neutralising Titer |
| | | | | env | gag | |
| 1 | **Regimen 1** | | | | | |
| | Ad7-env, Ad7-gag-1 Ad4-env, Ad4-gag-1 Ad5-env subunit: env + gag | 2,2 2,2 7+ | 128 8 128 | - - 100 100 | 20 20 - 1000 | <10 <10 <10 <10 |

| | **Regimen 2** | | | | | |
|---|---|---|---|---|---|---|
| | Ad7-env, Ad7-gag | 12 | 512 | 10000 | 10000 | 10 |

| 2 | **Regimen 1** | | | | | |
|---|---|---|---|---|---|---|
| | Ad7-env, Ad7-gag-1 Ad4-env, Ad4-gag-1 Ad5-env subunit: env + gag | 3,2 1,7 7+ | 64 128 64 | - 20 10000 1000 | 20 100 - 10000 | <10 <10 20 10 |

| | **Regimen 2** | | | | | |
|---|---|---|---|---|---|---|
| | Ad7-env, Ad7-gag | 9 | 8192 | 1000 | 10000 | 20 |

| 3 | **Regimen 1** | | | | | |
|---|---|---|---|---|---|---|
| | Ad7-env Ad4-env Ad5-env subunit: env | 2 1 7+ | 6 128 512 | 20 20 1000 100 | N/A* N/A N/A N/A | <10 <10 <10 <10 |

| | **Regimen 2** | | | | | |
|---|---|---|---|---|---|---|
| | 2 Ad7-env | 7 | 256 | 10000 | N/A | 10 |

| | | | | | | |
|---|---|---|---|---|---|---|
| *N/A = not applicable. | | | | | | |

### Measurement of Immunogenicity: Treatment Regimen 3

### Chimpanzee Inoculations and Collection of Data

Three chimpanzees (2 males and 1 female) that were screened negative for the presence of neutralizing antibodies to human adenoviruses type 4, 5, and 7 were evaluated using treatment regimen 3. Two chimpanzees (numbers 4 and 5) received both env and gag recombinant viruses while the third chimp (number 6) received only env recombinant viruses. Antibiotics were administered prophylactically to the chimpanzees and no respiratory disorders were observed.

Whole blood, serum, and stool samples were collected at different times during the course of the experiment, and processed as described in Regimen 1. Adenovirus detection in stool samples or nasal swabs, adenovirus neutralization assays, and detection of anti-HIV antibodies were performed according to the procedures described in Regimen 1.

### Results

**1st Immunization with Ad7-recombinants:** Recombinant viruses were shed into feces for 22 to 34 days post-infection. No recombinant viruses were detected in nasal secretions taken at 2 weeks post-infection. Seroconversion to the serotype of the adenovirus vectors employed was determined by neutralization assays. Excellent anti-adenovirus serum titers were measured in all 3 chimpanzees to Ad7 serotypes used in each of the chimpanzees. Seroconversion to recombinant HIV gene products were determined by Western blotting. Four weeks following the primary immunization with Ad7-recombinants anti-env and anti-gag responses could be measured in 2 of the 3 chimpanzees. By 20 weeks post-infection all 3 animals had measurable antibodies to HIV antigens. Secretory antibodies were not found in nasal swabs taken within the first 4 weeks following primary immunization. All 3 chimpanzees failed to mount detectable anti-HIV neutralizing antibody responses.

**1st Booster Immunization with Ad4-recombinants:** Recombinant Ad4 viruses were shed into feces for 14-28 days post-infection. Examination of nasal swabs indicated that recombinant Ad4 viruses could be detected in all 3 chimpanzees for at least 7 days post-infection. Significant anti-Ad4 responses were mounted against the Ad4 serotype following intranasal admininstration . The magnitude was slightly lower then that measured against the Ad7-recombinant viruses. Excellent booster responses to gag and/or env antigens were measured in all three animals. Low titered (1:2) anti-gag and/or anti-env responses were measured in nasal swabs from Chimpanzees 4 and 5. Still no anti-HIV neutralizing antibodies were measured in any of the animals.

**2nd Booster Immunization with Ad5-recombinants:** Recombinant Ad5 viruses were shed into feces for 8 days post-infection. No recombinant viruses could be detected in nasal swabs at 0, 1, or 2 weeks post-infection. Secretory IgG and IgA could be measured in nasal swabs taken from all 3 animals. Secretory antibodies were detected in the saliva collected from all 3 animals and from vaginal swabs taken from the one female chimpanzee. Anti-HIV type-specific neutralizing antibodies were detected in all three chimpanzees.

**Summary Tables:** The following table shows the results obtained using treatment regimen 3.

| **RESULTS OBTAINED USING TREATMENT REGIMEN 3** | | | | | | |
|---|---|---|---|---|---|---|
| Chimp Number | Recombinant Virus | Recombinant Virus Shedding Stools (Days) | Peak Anti-Adeno Neutralizing Titer | Western Blot Peak anti-HIV Titers | | Peak Anti-HIV Neutralizing Titer |
| | | | | env | gag | |
| 4 | Ad7-env, Ad7-gag Ad4-env, Ad4-gag Ad5-env, Ad5-gag subunit: env | 22,22 14,14 8,8 N/A* | 1024 128 32 N/A | 100 10000 10000 >10000 | 1000 10000 10000 10000 | <10 <10 20 640 |
| 5 | Ad7-env, Ad7-gag Ad4-env, Ad4-gag Ad5-env, Ad5-gag subunit: env | 34,27 14,14 8,8 N/A | 1024 512 32 N/A | 100 10000 10000 1000 | 10000 10000 10000 10000 | <10 <10 20 320 |
| 6 | Ad7-env Ad4-env Ad5-env subunit: env | 34 28 8 N/A | 1024 512 32 N/A | 100 10000 10000 >10000 | N/A N/A N/A N/A | <10 <10 40 320 |

| | | | | | | |
|---|---|---|---|---|---|---|
| *N/A = not applicable. | | | | | | |

The following table summarizes anti-HIV responses detected in chimpanzee secretions following intranasal booster immunization with the Ad5-HIV recombinants.

| **ANTI-HIV RESPONSES DETECTED IN SECRETIONS** | | | | | | | | |
|---|---|---|---|---|---|---|---|---|
| Chimp Number | Antigen Recognized | Weeks Post Boost | Secretion Analyzed | | | | | |
| | | | Nasal | | Saliva | | Vaginal | |
| | | | IgA | IgG | IgA | IgG | IgA | IgG |
| 4 | env | 0 1 2 4 | _* 180 180 720 | 360 360 2880 1440 | - - 20 - | - - 20 20 | - - - - | - 90 90 360 |
| | gag | 0 1 2 4 | - 360 720 720 | 180 360 2880 720 | - - - - | - 20 20 20 | - - - - | - 90 90 90 |
| 5 | env | 0 1 2 4 | - - - - | - 90 2880 360 | - - - - | - - - - | N/A⁺ N/A N/A N/A | N/A N/A N/A N/A |
| | gag | 0 1 2 4 | - - 90 - | - 90 1440 360 | - - - - | - - - - | N/A N/A N/A N/A | N/A N/A N/A N/A |
| 6 | env | 0 1 2 4 | - - - - | - - 1440 360 | - - 20 - | - - 20 - | N/A N/A N/A N/A | N/A N/A N/A N/A |

| | | | | | | | | |
|---|---|---|---|---|---|---|---|---|
| * - equals less than 90 for nasal and vaginal swabs and less than 20 for saliva samples. | | | | | | | | |
| ⁺ N/A = not applicable. | | | | | | | | |

The live recombinant adenovirus to be used according to the invention for the production of vaccines can be constructed by inserting into an adenovirus a gene coding for an antigen which corresponds to an antibody to an infectious organism in warm-blooded animals or which induces cell mediated immunity to an infectious organism in warm-blooded animals. The insertion may be carried out as described in P.K. Chanda et al., Virology, 175, 535-547 (1990), see particularly page 539.

The live recombinant adenovirus to be used according to the invention may be purified by placque purification. Quantities of the virus may be increased for vaccine production by replication in a suitable host. Replication may be carried out in cell culture. The cells of the cell culture are preferably BK (bovine kidney) cells or human carcinoma cells, particularly human cervical carcinoma cells for instance, the Hela cell line, or human lung carcinoma cells, for instance, the A549 cell line. The virus may be stored at -70°C in media such as DMEM with 5% fetal calf serum.

For intranasal administration the vaccine may comprise the virus with a stabilizer, for instance, SPGA (sucrose, phosphate, glutarate, and albumin) and, where desired, one or more sugars.

The invention is preferably used for the treatment of mammals of the order Primates (including man).

## Claims

1. Use of a live recombinant adenovirus to prepare a medicament for use in treatment to produce antibodies or cell mediated immunity to human immunity virus type 1, in which the virion structural protein of the live recombinant adenovirus is unchanged from that in the native adenovirus from which the recombinant adenovirus is produced, and which contains the gene coding for an antigen corresponding to the said antibodies or inducing the said cell mediated immunity, wherein the recombinant adenovirus is selected from one or more of the group consisting of
Ad7-tplenv-tplHrev, Ad7-tplgag-tplHrev, Ad7-rev-gag,
Ad4-tplenv-tplHrev, Ad4-tplgag-tplHrev, Ad4-rev-gag, Ad5-tplenv-tplHrev, and Ad5-tplgag-tplHrev, the medicament being for intranasal administration to a mammal of the order Primates including man.

2. Use as claimed in claim 1 which further comprises using one or more than one human immunodeficiency type 1 subunit protein to prepare a medicament for administration in the said method of producing antibodies or cell mediated immunity.

3. Use as claimed in claim 2, wherein the subunit is env or gag.

4. Use as claimed in claim 2 or 3, wherein one or more doses of the live recombinant adenovirus are to be administered with subsequent administration of one or more doses of a protein which is a subunit of human immunodeficiency type 1.

5. Use as claimed in any one of claims 2 to 4, wherein the protein which is a subunit of human immunodeficiency type 1 is env.

6. A live recombinant adenovirus which is X-tplY-tplHrev; wherein X is Ad4, Ad5, or Ad7 and Y is env or gag.

7. The recombinant adenovirus according to claim 6 which is Ad4-tplenv-tplHrev.

8. The recombinant adenovirus according to claim 6 which is Ad5-tplenv-tplHrev.

9. The recombinant adenovirus according to claim 6 which is Ad7-tplenv-tplHrev.

10. The recombinant adenovirus according to claim 6 which is Ad4-tplgag-tplHrev.

11. The recombinant adenovirus according to claim 6 which is Ad5-tplgag-tplHrev.

12. The recombinant adenovirus according to claim 6 which is Ad7-tplgag-tplHrev.

## Patentansprüche

1. Verwendung eines lebenden rekombinanten Adenovirus zur Herstellung eines Medikaments zur Verwendung bei einer Behandlung zur Erzeugung von Antikörpern oder einer zellvermittelten Immunität gegen menschliche―Immunität―Typ―1―Viren, worin das Strukturprotein des Virions des lebenden rekombinanten Adenovirus unverändert gegenüber jenem im nativen Adenovirus ist, aus dem das rekombinante Adenovirus erzeugt ist und welches das Gen enthält, das für ein Antigen codiert, das besagten Antikörpern entspricht oder besagte zellvermittelte Immunität induziert, wobei das rekombinante Adenovirus ausgewählt ist aus einem oder mehr der Gruppe bestehend aus Ad7-tplenv-tplHrev, Ad7-tplgag-tplHrev, Ad7-rev-gag, Ad4-tplenv-tplHrev, Ad4-tplgag-tplHrev, Ad4-rev-gag, Ad5-tplenv-tplHrev und Ad5-tplgagtplHrev, welches Medikament für die intranasale Verabreichung an einen Säuger der Ordnung Primaten einschließlich des Menschen bestimmt ist.

2. Verwendung nach Anspruch 1, welche weiters die Verwendung von einem oder mehr menschlichen Immunschwäche Typ 1-Untereinheit-Protein(en) zur Herstellung eines Medikaments zur Verabreichung beim Verfahren zur Herstellung von Antikörpern oder zellvermittelter Immunität umfasst.

3. Verwendung nach Anspruch 2, worin die Untereinheit env oder gag ist.

4. Verwendung nach Anspruch 2 oder 3, worin eine oder mehr Dosen des lebenden rekombinanten Adenovirus zu verabreichen sind bei anschließender Verabreichung von einer oder mehr Dosen eines Proteins, das eine menschliche Immunschwäche Typ 1-Untereinheit ist.

5. Verwendung nach einem der Ansprüche 2 bis 4, worin das Protein, das eine menschliche Immunschwäche Typ 1-Untereinheit ist, env ist.

6. Lebendes rekombinantes Adenovirus, der X-tp1Y-tp1Hrev ist, worin X Ad4, Ad5 oder Ad7 ist und Y env oder gag ist.

7. Rekombinantes Adenovirus nach Anspruch 6, das Ad4-tplenv-tplHrev ist.

8. Rekombinantes Adenovirus nach Anspruch 6, das Ad5―tplenv―tp1Hrev ist.

9. Rekombinantes Adenovirus nach Anspruch 6, das Ad7―tplenv―tp1Hrev ist.

10. Rekombinantes Adenovirus nach Anspruch 6, das Ad4-tplgag-tplHrev ist.

11. Rekombinantes Adenovirus nach Anspruch 6, das Ad5―tplgag―tplHrev ist.

12. Rekombinantes Adenovirus nach Anspruch 6, das Ad7―tplgag―tp1Hrev ist.

## Revendications

1. Utilisation d'un adénovirus recombiné vivant pour préparer un médicament pour l'utilisation dans le traitement pour produire des anticorps ou l'immunité à médiation cellulaire au virus de l'immunité humaine de type 1, dans laquelle la protéine de structure du virion de l'adénovirus recombiné vivant est inchangée par rapport à celle de l'adénovirus natif à partir duquel l'adénovirus recombiné est produit, et qui contient le gène codant pour un antigène correspondant auxdits anticorps ou induisant ladite immunité à médiation cellulaire, dans laquelle l'adénovirus recombiné est choisi parmi un ou plusieurs du groupe constitué de : Ad7-tplenv-tplHrev, Ad7-tplgag-tplHrev, Ad7-rev-gag, Ad4-tplenv-tplHrev, Ad4-tplgag-tplHrev, Ad4-rev-gag, Ad5-tplenv-tplHrev et Ad5-tplgag-tplHrev, le médicament étant destiné à l'administration intranasale à un mammifère de l'ordre des primates y compris l'homme.

2. Utilisation selon la revendication 1 qui comprend en outre l'utilisation d'une ou de plus d'une protéine sous-unitaire du déficit immunitaire humain de type 1 pour préparer un médicament pour l'administration dans ledit procédé de production des anticorps ou de l'immunité à médiation cellulaire.

3. Utilisation selon la revendication 2 dans laquelle la sous-unité est env ou gag.

4. Utilisation selon la revendication 2 ou 3, dans laquelle une ou plusieurs doses de l'adénovirus recombiné vivant doivent être administrées avec l'administration subséquente d'une ou plusieurs doses d'une protéine qui est une sous-unité du déficit immunitaire humain de type 1.

5. Utilisation selon l'une quelconque des revendications 2 à 4 dans laquelle la protéine qui est une sous-unité du déficit immunitaire humain de type 1 est env.

6. Adénovirus recombiné vivant qui est X-tplY-tplHrev ; dans lequel X est Ad4, Ad5 ou Ad7 et Y est env ou gag.

7. Adénovirus recombiné selon la revendication 6 qui est Ad4-tplenv-tplHrev.

8. Adénovirus recombiné selon la revendication 6 qui est Ad5-tplenv-tplHrev.

9. Adénovirus recombiné selon la revendication 6 qui est Ad7-tplenv-tplHrev.

10. Adénovirus recombiné selon la revendication 6 qui est Ad4-tplgag-tplHrev.

11. Adénovirus recombiné selon la revendication 6 qui est Ad5-tplgag-tplHrev.

12. Adénovirus recombiné selon la revendication 6 qui est Ad7-tplgag-tplHrev.
